# EUROPEAN PATENT APPLICATION

(11) **EP 1 525 892 A2**
(43) Date of publication of application: **27.04.2005**
(21) Application number: 04025182.9
(22) Date of filing: 22.10.2004
(51) Int. Cl.: A61M 1/10

(54) **Artificial blood vessel system, connecting assist tool and blood pump system**

(30) Priority: 24.10.2003 JP 2003364186
(71) Applicant: SUN MEDICAL TECHNOLOGY RESEARCH CORPORATION, Suwa-shi, Nagano (JP)
(72) Inventor: Yamazaki, Kenji, Koganei-shi Tokyo (JP); Kitano, Tomoya, Suwa-shi Nagano (JP); Nakayama, Takeshi, Suwa-shi Nagano (JP)
(74) Representative: Leson, Thomas Johannes Alois, Dipl.-Ing.

(57) **Abstract**

The present invention provides an artificial blood vessel system which can suppress the occurrence of the stagnation of a blood flow and, eventually, the occurrence of the thrombus, can enhance the reliability of the connection, and can prolong the lifetime of the artificial blood connecting structure. In the artificial blood vessel system which includes an artificial blood vessel, a tubular connecting member which is connected with the artificial blood vessel, and connecting assist means for strengthening the connection between the artificial blood vessel and the tubular connecting member, the connecting assist means is configured to be capable of bringing the artificial blood vessel into pressure contact with the tubular connecting member not only along the radial direction of the tubular connecting member but also at an artificial-blood-vessel-side distal end of the tubular connecting member.

## Description

### BACKGROUND OF THE INVENTION

### 1. Field of the Invention

The present invention relates to an artificial blood vessel system, a connecting assist tool and a blood pump system.

### 2. Related Art

As one of methods for curing a serious heart disease, a medical treatment using a blood pump has been performed. To perform such a medical treatment, it is necessary to connect the blood pump and a heart as well as the blood pump and an aorta respectively using artificial blood vessels. Accordingly, various artificial blood vessel connecting structures are used at portions where these parts and organs are connected (see, for example, German Laid-open Patent Publication 10108813A1 (Fig. 1 to Fig. 3)).

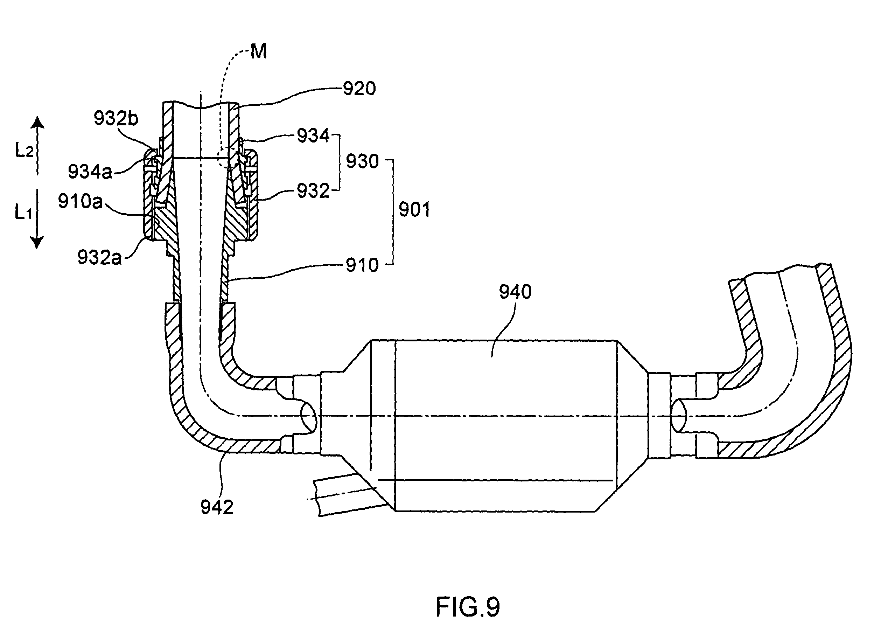

Fig. 9 is a cross-sectional view showing the artificial blood vessel connecting structure which is described in the above-mentioned patent publication. This artificial blood vessel connecting structure 901 is, as shown in Fig. 9, an artificial blood vessel connecting structure which is served for connecting a blood pump 940 and an artificial blood vessel 920. The artificial blood vessel connecting structure 901 includes a tubular connecting member 910 which is connected with the blood pump 940 by way of a bend 942 and has a male threaded portion 910a, the artificial blood vessel 920 which is connected with an opening end portion on a side opposite to the blood pump 940 out of both opening end portions of the tubular connecting member 910, and a connecting assist tool 930 which is served for strengthening the connection between the artificial blood vessel 920 and the tubular connecting member 910.

The connecting assist tool 930 is constituted of a nut 932 and a pressure ring 934. The nut 932 includes a female threaded portion 932a and a flange 932b and the female threaded portion 932a of the nut 932 is threadedly engaged with the male threaded portion 910a of the tubular connecting member 910. The pressure ring 934 includes a flange 934a which is interposed between an outer peripheral surface of the artificial blood vessel 920 and the nut 932.

In such an artificial blood vessel connecting structure 901, in connecting the artificial blood vessel 920 with the tubular connecting member 910, first of all, the connecting assist tool 930 is mounted on a periphery of the artificial blood vessel 920. Then, the artificial blood vessel 920 in this state is fitted on and connected with the tubular connecting member 910 such that the artificial blood vessel 920 covers a distal end portion of the tubular connecting member 910. Thereafter, the female threaded portion 932a of the nut 932 of the connecting assist tool 930 is threadedly engaged with the male threaded portion 910a of the tubular connecting member 910. Here, in advancing the nut 932 in the L₁ direction in Fig. 9 by performing the rotational manipulation of the nut 932, the flange 932b of the nut 932 is brought into contact with pressure contact with the flange 934a of the pressure ring 934 in the L₁ direction in Fig. 9. In such an artificial blood vessel connecting structure 901, a profile of the tubular connecting member 910 is gradually increased in the L₁ direction in Fig. 9 and hence, the artificial blood vessel 920 is brought into pressure contact with the tubular connecting member 910 due to this pressure contact force. Accordingly, the artificial blood vessel 920 and the tubular connecting member 910 are firmly connected with each other.

### SUMMARY OF THE INVENTION

However, in such an artificial blood vessel connecting structure 901, the artificial blood vessel 920 and the tubular connecting member 910 are connected with each other by making use of a so-called wedge effect. Accordingly, a contact pressure force component along the radial direction of the tubular connecting member 910 out of a contact pressure force applied to the artificial blood vessel 920 from the pressure ring 934 is gradually decreased from the blood-pump-side end portion (connection terminal end portion) to the artificial-blood-vessel-side end portion (connection start end portion) (along the L₂ direction in Fig. 9).

Accordingly, at the artificial-blood-vessel-side distal end (indicated by symbol M in Fig. 9) of the tubular connecting member 910, the pressure contact of the artificial blood vessel 920 with the tubular connecting member 910 is not guaranteed. As a result, there arises a possibility that a minute gap is formed between the tubular connecting member 910 and the artificial blood vessel 920 at the artificial-blood-vessel-side distal end (M) of the tubular connecting member 910. When the artificial blood vessel is flexible, the possibility that such a gap is formed is particularly increased. Once such a gap is formed, due to the presence of the gap, a blood flow is stagnated thus giving rise to a drawback that there exists a possibility of the occurrence of the thrombus.

Further, in the artificial blood vessel connecting structure 901, since the nut 932 and the pressure ring 934 are brought into contact with each other only in the axial direction at both flanges 932b, 934a portions and are not brought into contact with each other in the radial direction and hence, there exists a play in the radial direction between the nut 932 and the pressure ring 934.

Accordingly, when the artificial blood vessel 920 is bent in the vicinity of the artificial-blood-vessel-side distal end (M) of the tubular connecting member 910, the pressure ring 934 assumes an eccentric position with respect to the tubular connecting member 910 and hence, there arises a possibility that a minute gap is formed between the artificial blood vessel 920 and the tubular connecting member 910. When the gap is formed, in the same manner as the above-mentioned case, the blood flow is stagnated due to the presence of the gap thus giving rise to a drawback that there exists a possibility of the occurrence of the thrombus.

Further, in this artificial blood vessel connecting structure 901, the artificial blood vessel 920 and the tubular connecting member 910 are connected with each other while turning around the pressure ring 934 and hence, an undesired stress attributed to twisting or the like is applied to the artificial blood vessel 920. Accordingly, the connection of the artificial blood vessel is liable to easily become non-uniform thus lowering the reliability of the connection between the artificial blood vessel 920 and the tubular connecting member 910 in the artificial blood vessel connecting structure 901. Further, there has been also a drawback that the artificial blood vessel 920 is easily damaged and hence, the lifetime of the artificial blood vessel connecting structure 901 is shortened. Still further, this undesired stress attributed to twisting or the like is transmitted to a portion where the artificial blood vessel is connected with a living tissue anastomosis portion, the blood pump and the like and imparts an adverse effect on the portion.

The present invention has been made to overcome such drawbacks and it is an object of the present invention to provide an artificial blood vessel system and a blood pump system which include the artificial blood vessel connecting structure which can suppress the stagnation of a blood flow and, eventually, the occurrence of the thrombus and can prolong the lifetime of the artificial blood vessel connecting structure without lowering the reliability of the connection.

It is another object of the present invention to provide a connecting assist tool which can be suitably used in such an artificial blood vessel system.
(1) An artificial blood vessel system according to the present invention includes an artificial blood vessel, a tubular connecting member which is connected with the artificial blood vessel, and connecting assist means for strengthening the connection between the artificial blood vessel and the tubular connecting member, wherein the connecting assist means is configured to be capable of bringing the artificial blood vessel into pressure contact with the tubular connecting member not only along the radial direction of the tubular connecting member but also at an artificial-blood-vessel-side distal end of the tubular connecting member.
   Due to such a constitution, according to the artificial blood vessel system of the present invention, the connecting assist means can bring the artificial blood vessel into pressure contact with the tubular connecting member along the radial direction of the tubular connecting member and hence, an undesired stress attributed to twisting or the like is not applied to the artificial blood vessel. As a result, the connection of the artificial blood vessel can be performed more uniformly and hence, the reliability of connection between the artificial blood vessel and the tubular connecting member in the artificial blood vessel system can be enhanced. Further, damage to the artificial blood vessel can be effectively prevented and hence, the lifetime of the artificial blood vessel system can be prolonged. Further, it is possible to suppress any adverse influence to a part such as a living tissue anastomosis portion, a blood pump or the like to which the artificial blood vessel system is connected.
   Further, according to the artificial blood vessel system of the present invention, the connecting assist means can bring the artificial blood vessel into pressure contact with the tubular connecting member also at the artificial-blood-vessel-side distal end of the tubular connecting member and hence, it is possible to effectively prevent the formation of a minute gap between the artificial blood vessel and the tubular connecting member whereby it is possible to effectively prevent the occurrence of the stagnation of the blood flow and, eventually, the occurrence of the thrombus.
   In this case, it is considered that, when the artificial blood vessel is bent in the vicinity of the artificial-blood-vessel-side distal end of the tubular connecting member, a minute gap is formed between the artificial blood vessel and the tubular connecting member and eventually the blood flow is stagnated thus considerably increasing the possibility of the occurrence of the thrombus. However, even in such a case, according to the artificial blood vessel system of the present invention, the artificial blood vessel is brought into pressure contact with the tubular connecting member also at the artificial-blood-vessel-side distal end of the tubular connecting member and hence, the formation of the minute gap between the artificial blood vessel and the tubular connecting member can be effectively suppressed. As a result, it is possible to effectively prevent the occurrence of the stagnation of the blood flow and, eventually, the occurrence of the thrombus.
   Here, in the artificial blood vessel system of the present invention, by using the connecting assist means which brings the artificial blood vessel into pressure contact with the tubular connecting member in the radial direction of the tubular connecting member at the artificial-blood-vessel-side distal end of the tubular connecting member as the connecting assist means, it is possible to obtain the substantially equal advantageous effects.
(2) In the artificial blood vessel system having the above-mentioned constitution (1), it is preferable that the connecting assist means has the property of narrowing an inner diameter thereof.
   Due to such a constitution, it is possible to bring the artificial blood vessel into pressure contact with the tubular connecting member more uniformly and hence, it is possible to further effectively prevent the applying of the undesired stress attributed to twisting or the like to the artificial blood vessel.
   In this case, as the connecting assist means which has the property of narrowing the inner diameter thereof, a winding member which applies a fastening force in the direction to make an inner diameter thereof small, a heat-shrinkable tube which is liable to shrink in the direction to make an inner diameter thereof small, a shape memory alloy which is liable to shrink in the direction to make an inner diameter thereof small, a resilient member such as a tube or a ring made of silicone rubber, a tube or a ring made of fluoric rubber can be exemplified. Further, connecting assist means which may be wound around with a tie band, strings or wires may be also used as the connecting assist means.
(3) In the artificial blood vessel system having the above-mentioned constitution (1) or (2), it is preferable that the connecting assist means is constituted of a connecting assist tool which satisfies a following relationship (a):
   (a) the relationship "Za<Zc and Zb-Zc≥-2×t", wherein assuming an imaginary axis which sets the direction heading for the artificial blood vessel from the tubular connecting member as the normal direction as a z axis, Za are coordinates of the z axis at a tubular-connecting -member-side end of the connecting assist tool, Zb are coordinates of the z axis at an artificial-blood-vessel-side end of the connecting assist tool, Zc are coordinates of the z axis at an artificial-blood-vessel-side distal end of the tubular connecting member, and "t" is a wall thickness of an unconnected portion of the artificial blood vessel.
      In the artificial blood vessel system of the present invention, to surely bring the artificial blood vessel into pressure contact with the tubular connecting member even at the artificial-blood-vessel-side distal end of the tubular connecting member, it is preferable that the position of the artificial-blood-vessel-side end of the connecting assist means is arranged closer to the artificial-blood-vessel side than the artificial-blood-vessel-side distal end of the tubular connecting member.
      However, according to a result of an experiment carried out by inventors of the present invention, it has been found that even when the position of the artificial-blood-vessel-side end of the connecting assist means is arranged closer to the tubular-connecting-member side than the artificial-blood-vessel-side distal end of the tubular connecting member, with the use of the connecting assist tool which satisfies the relationship "Za<Zc and Zb-Zc≥-2×t", the connecting assist tool can bring the artificial blood vessel into pressure contact with the tubular connecting member with a sufficient force even at the artificial-blood-vessel-side distal end of the tubular connecting member and hence, the occurrence of the minute gap between the artificial blood vessel and the tubular connecting member can be suppressed whereby it is possible to effectively suppress the occurrence of the stagnation of the blood flow and, eventually, the occurrence of the thrombus. It is estimated that the artificial blood vessel is brought into pressure contact with the tubular connecting member with a greater force due to the resiliency of the artificial blood vessel.
      Here, as the artificial blood vessel, it is preferable to use an artificial blood vessel having a wall thickness "t" of 0.1 mm to 2.5mm.
      However, to allow the connecting assist tool to bring the artificial blood vessel into pressure contact with the tubular connecting member with a sufficient force even at the artificial-blood-vessel-side distal end of the tubular connecting member thus further effectively suppressing the occurrence of the stagnation of the blood flow and, eventually, the occurrence of the thrombus, it is desirable that a relationship "Zb-Zc≥-t" is satisfied. It is further desirable that a relationship "Zb-Zc≥-0.5×t" is satisfied. It is still further desirable that a relationship "Zb-Zc≥0mm" is satisfied.
      On the other hand, when the value of "Zb-Zc" is excessively large, a range within which the artificial blood vessel is not bent is increased and hence, the degree of freedom at the time of embedding the artificial blood vessel into a living body becomes low. From this point of view, it is preferable that a relationship "Zb-Zc≤20×t" is satisfied. It is more preferable that a relationship "Zb-Zc≤10×t" is satisfied. It is still more preferable that a relationship "Zb-Zc≤5×t" is satisfied.
(4) In the artificial blood vessel system having the above-mentioned constitution (3), it is preferable that a member which prevents the artificial blood vessel from being sharply bent is mounted on an outer periphery of the artificial blood vessel.
   As an artificial blood vessel, there has been known an artificial blood vessel which winds a resilient member called "auxiliary helix" on an outer periphery thereof as a member which prevents the artificial blood vessel from being sharply bent. When the artificial blood vessel which winds the auxiliary helix thereon is used in the artificial blood vessel system, it is possible to easily maintain an open state of the artificial blood vessel due to the resiliency of the auxiliary helix and hence, it is possible to suppress the sharp bending of the artificial blood vessel.
   Here, as the member which prevents the sharp bending of the artificial blood vessel, besides the auxiliary helix, the structure which arranges ring-like members on an outer periphery of the artificial blood vessel at a given interval or the structure which arranges a mesh-like member on an outer periphery of the artificial blood vessel can be exemplified.
   Here, it is preferable that the member which is provided for preventing the sharp bending of the artificial blood vessel is brought into contact with the connecting assist tool. Due to such a constitution, it is possible to suppress the concentration of a stress on the artificial blood vessel at the artificial-blood-vessel-side distal end of the tubular connecting member and hence, buckling and wrinkles are hardly generated whereby the flow of the blood is hardly impeded and the thrombus hardly occurs.
(5) In the artificial blood vessel system having the above-mentioned constitution (1) or (2), it is preferable that a member for preventing the artificial blood vessel from being sharply bent is mounted on an outer periphery of the artificial blood vessel, and the connecting assist means is constituted of a connecting assist tool which satisfies a following relationship (b) and the member for preventing the artificial blood vessel from being sharply bent, and the tubular-connecting-member-side end of the member for preventing the artificial blood vessel from being sharply bent is brought into contact with the artificial-blood-vessel-side end of the connecting assist tool:
   (b) the relationship "Zb<Zc", wherein assuming an imaginary axis which sets the direction heading for the artificial blood vessel from the tubular connecting member as the normal direction as a z axis, Zb are coordinates of the z axis at an artificial-blood-vessel-side end of the connecting assist tool, and Zc are coordinates of the z axis at an artificial-blood-vessel-side distal end of the tubular connecting member.

   As mentioned above, as an artificial blood vessel, there has been known an artificial blood vessel which winds a resilient member called "auxiliary helix" on an outer periphery thereof as a member for preventing the artificial blood vessel from being sharply bent. When the artificial blood vessel which winds the auxiliary helix thereon is used in the artificial blood vessel system, by bringing the tubular-connecting-member-side end of the auxiliary helix into contact with the artificial-blood-vessel-side end of the connecting assist tool, the auxiliary helix brings the artificial blood vessel into pressure contact with the tubular connecting member even at the artificial-blood-vessel-side distal end of the tubular connecting member and hence, the formation of a minute gap between the artificial blood vessel and the tubular connecting member can be suppressed whereby it is possible to effectively suppress the occurrence of the stagnation of the blood flow and, eventually, the occurrence of the thrombus. The same goes for the case in which a member other than the auxiliary helix is used as a member for preventing the sharp bending of the artificial blood vessel.
(6) In the artificial blood vessel system having any one of the above-mentioned constitutions (1) to (5), it is preferable that a relationship "ID₁≥ ID₀" is satisfied wherein ID₀ is an inner diameter of an unconnected portion of the artificial blood vessel and ID₁ is an inner diameter of the artificial blood vessel at an artificial-blood-vessel-side distal end of the tubular connecting member.
   Due to such a constitution, at the artificial-blood-vessel-side distal end of the tubular connecting member, the artificial blood vessel is brought into pressure contact with the tubular connecting member even with a resilient force of the artificial blood vessel per se and hence, it is possible to further effectively suppress the occurrence of the stagnation of the blood flow and, eventually, the occurrence of the thrombus.
   In this case, It is more preferable that the relationship "ID₁>ID₀" is satisfied. This is because that the artificial blood vessel is brought into pressure contact with the tubular connecting member with a greater force due to the resilient force of the artificial blood vessel per se.
(7) In the artificial blood vessel system having the above-mentioned constitution (6), it is preferable that a relationship "ID₁/ID₀<1+0.11×t^{-1/2}" is satisfied wherein t(mm) is a wall thickness of an unconnected portion of the artificial blood vessel.
   When the artificial blood vessel system satisfies such a relationship, the bending of the artificial blood vessel in the vicinity of the artificial-blood-vessel-side distal end of the tubular connecting member can be made gentle and hence, it is possible to suppress the occurrence of the stagnation of the blood flow and, eventually, the occurrence of the thrombus. Here, this relationship is an empirical relationship which is introduced by an experiment carried out by inventors of the present invention and does not always have the physical implication.
(8) In the artificial blood vessel system having any one of the above-mentioned constitutions (1) to (7), it is preferable that relationships "OD₁>OD₀" and "OD₁>OD₂" are satisfied wherein OD₀ is an outer diameter of an unconnected portion of the artificial blood vessel, OD₁ is an outer diameter of a portion which is not subjected to a contact pressure force attributed to the connecting assist means out of portions of the artificial blood vessel which is applied to an outer periphery of the tubular connecting member, and OD₂ is an outer diameter of a portion which is subjected to the contact pressure force attributed to the connecting assist means out of the portions of the artificial blood vessel which is applied to the outer periphery of the tubular connecting member.
   Due to such a constitution, it is guaranteed that the pressure contact force exerted by the connecting assist means is surely applied to the artificial blood vessel. Accordingly, even at the artificial-blood-vessel-side distal end of the tubular connecting member, the artificial blood vessel is surely brought into pressure contact with the tubular connecting member and hence, the formation of a minute gap between the artificial blood vessel and the tubular connecting member can be further effectively suppressed whereby the occurrence of the stagnation of the blood flow and, eventually, the occurrence of the thrombus can be further effectively suppressed.
(9) In the artificial blood vessel system having any one of the above-mentioned constitutions (1) to (8), it is preferable that, on the artificial-blood-vessel-side end portion of the tubular connecting member, a sharpened end portion whose wall thickness is gradually decreased from the tubular-connecting-member side to the artificial-blood-vessel side is formed.
   Due to such a constitution, the flow of the blood at a portion where the artificial blood vessel and the tubular connecting member are connected with each other can be made further smoother and hence, it is possible to further effectively suppress the occurrence of the stagnation of the blood flow and, eventually, the occurrence of the thrombus. Further, it is also possible to obtain an advantageous effect that the assembling operation at the time of connecting the artificial blood vessel to the tubular connecting member is facilitated.
(10) In the artificial blood vessel system having the above-mentioned constitution (9), it is preferable that the rounding treatment is applied to the sharpened end portion of the tubular connecting member.
   Due to such a constitution, a load applied to the artificial blood vessel from the sharpened end portion of the tubular connecting member can be alleviated and hence, an undesired biting of the sharpened end portion of the tubular connecting member into the artificial blood vessel can be obviated. Accordingly, damage to the artificial blood vessel can be effectively suppressed and hence, the lifetime of the artificial blood vessel system can be further prolonged. Further, it is possible to further effectively suppress the occurrence of the stagnation of the blood flow and, eventually, the occurrence of the thrombus at the artificial-blood-vessel-side distal end of the tubular connecting member.
(11) In the artificial blood vessel system having any one of the above-mentioned constitutions (1) to (10), it is preferable that an uneven portion is formed on an outer peripheral surface of the tubular connecting member.
   Due to such a constitution, the artificial blood vessel and the tubular connecting member are connected with each other by way of the uneven portion. Accordingly, it is possible to realize the firm connection between the artificial blood vessel and the tubular connecting member whereby the occurrence of slipping of the artificial blood vessel from the tubular connecting member can be effectively prevented.
(12) In the artificial blood vessel system having any one of the above-mentioned constitutions (3) to (5), it is preferable that the connecting assist tool is a connecting assist tool which includes a fastening member arranged on an outer peripheral surface of the artificial blood vessel by way of a receiving member, and the connecting assist tool is arranged on an outer peripheral surface of the artificial blood vessel in a state that a fastening force of the fastening member is applied to the connecting assist tool.
   Due to such a constitution, the fastening force of the fastening member is transmitted along the radial direction of the tubular connecting member by way of the receiving member. Accordingly, the connecting assist tool brings the artificial blood vessel into pressure contact with the tubular connecting member along the radial direction of the tubular connecting member and hence, an undesired stress attributed to twisting or the like is not applied to the artificial blood vessel. As a result, the connection of the artificial blood vessel can be made more uniform and hence, the reliability of the connection between the artificial blood vessel and the tubular connecting member can be further enhanced. Further, damage to the artificial blood vessel can be effectively suppressed and hence, it is possible to further prolong the lifetime of the artificial blood vessel system. Still further, it is possible to suppress any adverse influence to a part such as a living tissue anastomosis portion, a blood pump or the like to which the artificial blood vessel system is connected.
   In this case, as the fastening member, a winding member which applies a fastening force in the direction which makes an inner diameter thereof small, a heat-shrinkable tube which is liable to shrink in the direction to make an inner diameter thereof small, a shape memory alloy which is liable to shrink in the direction to make an inner diameter thereof small, a resilient member such as a tube or a ring made of silicone rubber, a tube or a ring made of fluoric rubber can be exemplified. Further, a fastening member which may be wound around with a tie band, strings or wires may be also used as the fastening member.
(13) In the artificial blood vessel system having any one the above-mentioned constitutions (1) to (12), it is preferable that the artificial blood vessel is made of a material which exhibits a favorable blood compatibility.
   Due to such a constitution, it is possible to suppress the occurrence of the thrombus in the inside of the artificial blood vessel. As the material having the favorable blood compatibility, fluoric resin, polyurethane resin, polyester resin or the like can be used. However, from a viewpoint of the blood compatibility, the resiliency and the like, it is particularly preferable to use expanded polytetrafluoroethylene.
(14) In the artificial blood vessel system having any one of the above-mentioned constitutions (1) to (13), it is preferable that the tubular connecting member is made of a material which exhibits a favorable blood compatibility.
   Due to such a constitution, it is possible to suppress the occurrence of the thrombus in the inside of the tubular connecting member. As the material which exhibits the favorable blood compatibility, it is preferable to use pure titanium or titanium alloy. As the titanium alloy, it is preferable to use Ti-6Al-4V alloy (particularly, alloy of ELI (Extra Low Interstitial) grade) which is obtained by adding 6% of aluminum and 4% of vanadium to titanium.
(15) In the artificial blood vessel system having any one of the above-mentioned constitutions (1) to (14), it is preferable that, on a blood contact surface of the artificial blood vessel system, a coating film made of a material having blood compatibility and antithrombogenicity is formed.
   Due to such a constitution, it is possible to suppress the occurrence of the thrombus on the blood contact surface of the artificial blood vessel system.
   Here, as the material which possesses the blood compatibility and the antithrombogenicity, phospholipid polymer can be preferably used.
(16) In the artificial blood vessel system having any one of the above-mentioned constitutions (1) to (15), it is preferable that the artificial blood vessel system further includes a connecting ring for connecting the artificial blood vessel to the blood pump.
   Due to such a constitution, it is possible to easily connect the excellent artificial blood vessel system which has the prolonged lifetime, exhibits the high reliability in connection and can effectively suppress the occurrence of the thrombus with the blood pump.
(17) In the artificial blood vessel system having any one of the above-mentioned constitutions (1) to (16), it is preferable that the artificial blood vessel system further includes a cannula for connecting the artificial blood vessel to the heart.
   Due to such a constitution, it is possible to connect the excellent artificial blood vessel system which has the prolonged lifetime, exhibits the high reliability in connection and can effectively suppress the occurrence of the thrombus with the heart.
(18) A connecting assist tool of the present invention is a connecting assist tool for strengthening the connection between a tubular connecting member and an artificial blood vessel, wherein the connecting assist tool is served for the artificial blood vessel system having the above-mentioned constitution (3), (4), (5) or (12).
   Accordingly, by connecting the artificial blood vessel to the tubular connecting member using the connecting assist tool of the present invention, the connecting assist tool can bring the artificial blood vessel into pressure contact with the tubular connecting member along the radial direction of the tubular connecting member and hence, an undesired stress attributed to twisting or the like is not applied to the artificial blood vessel. As a result, the connection of the artificial blood vessel can be made more uniform and hence, the reliability of connection between the artificial blood vessel and the tubular connecting member can be enhanced. Further, damage to the artificial blood vessel can be effectively suppressed and hence, the lifetime of the artificial blood vessel system can be prolonged.
   Further, by connecting the artificial blood vessel to the tubular connecting member using the connecting assist tool of the present invention, the connecting assist tool can bring the artificial blood vessel into pressure contact with the tubular connecting member even at the artificial-blood-vessel-side distal end of the tubular connecting member and hence, the formation of a minute gap between the artificial blood vessel and the tubular connecting member can be effectively suppressed. As a result, it is possible to effectively suppress the occurrence of the stagnation of the blood flow and, eventually, the occurrence of the thrombus.
   In this case, it is considered that, when the artificial blood vessel is bent in the vicinity of the artificial-blood-vessel-side distal end of the tubular connecting member, a minute gap is formed between the artificial blood vessel and the tubular connecting member and eventually the blood flow is stagnated thus considerably increasing the possibility of the occurrence of the thrombus. However, even in such a case, with use of the connecting assist tool of the present invention, the connecting assist tool can bring the artificial blood vessel into pressure contact with the tubular connecting member also at the artificial-blood-vessel-side distal end of the tubular connecting member and hence, the formation of the minute gap between the artificial blood vessel and the tubular connecting member can be effectively suppressed. As a result, it is possible to effectively prevent the occurrence of the stagnation of the blood flow and, eventually, the occurrence of the thrombus.
(19) A blood pump system of the present invention is a blood pump system which includes a blood pump and an artificial blood vessel system which is connected to at least one side out of a suction side and a discharge side of the blood pump, wherein the artificial blood vessel system is the artificial blood vessel system having the above-mentioned constitution (16).
   Accordingly, the blood pump system of the present invention is provided with the artificial blood vessel system which exhibits the prolonged lifetime, has high reliability in connection and can effectively suppress the occurrence of the thrombus and, at the same time, can be easily connected with the blood pump and hence, it is possible to provide the highly reliable and excellent blood pump system which can reduce a burden imposed on an operator at the time of embedding the blood pump into a living body and can suppress the occurrence of the thrombus after embedding the blood pump into the living body.
(20) A blood pump system of the present invention is a blood pump system which comprises a blood pump which includes a tubular connecting member having at least one side out of a suction side and a discharge side thereof, an artificial blood vessel which is connected with the tubular connecting member, and connecting assist means which is served for strengthening the connection between the artificial blood vessel and the tubular connecting member, wherein the connecting assist means is configured to be capable of bringing the artificial blood vessel into pressure contact with the tubular connecting member not only in the radial direction of the tubular connecting member but also at an artificial-blood-vessel-side distal end of the tubular connecting member.
   Accordingly, the blood pump system of the present invention is provided with the artificial blood vessel system which exhibits the prolonged lifetime, has high reliability in connection and can effectively suppress the occurrence of the thrombus and hence, it is possible to provide the highly reliable and excellent blood pump system which can suppress the occurrence of the thrombus.

### BRIEF DESCRIPTION OF THE DRAWINGS

Fig. 1 is a view for explaining a blood pump system including an artificial blood vessel system according to an embodiment of the present invention;
Fig. 2 is a cross-sectional view showing a blood pump used in the blood pump system shown in Fig. 1;
Fig. 3 is a view for explaining the artificial blood vessel system according to the embodiment 1 of the present invention;
Fig. 4A and Fig. 4B are views for explaining the artificial blood vessel connecting structure 100A shown in Fig. 3;
Fig. 5A and Fig. 5B are views for explaining the artificial blood vessel connecting structure 100B shown in Fig. 3;
Fig. 6 is a view for explaining an artificial blood vessel system according to an embodiment 2 of the present invention;
Fig. 7 is a view for explaining an artificial blood vessel system according to an embodiment 3 of the present invention;
Fig. 8 is a view showing the artificial blood vessel connecting structure which is used in the blood pump system according to an embodiment 4 of the present invention; and
Fig. 9 is a cross-sectional view showing the conventional artificial blood vessel connecting structure.

### DESCRIPTION OF THE PREFERRED EMBODIMENTS

An artificial blood vessel system, a connecting assist tool and a blood pump system to which the present invention is applied are explained hereinafter in conjunction with drawings.

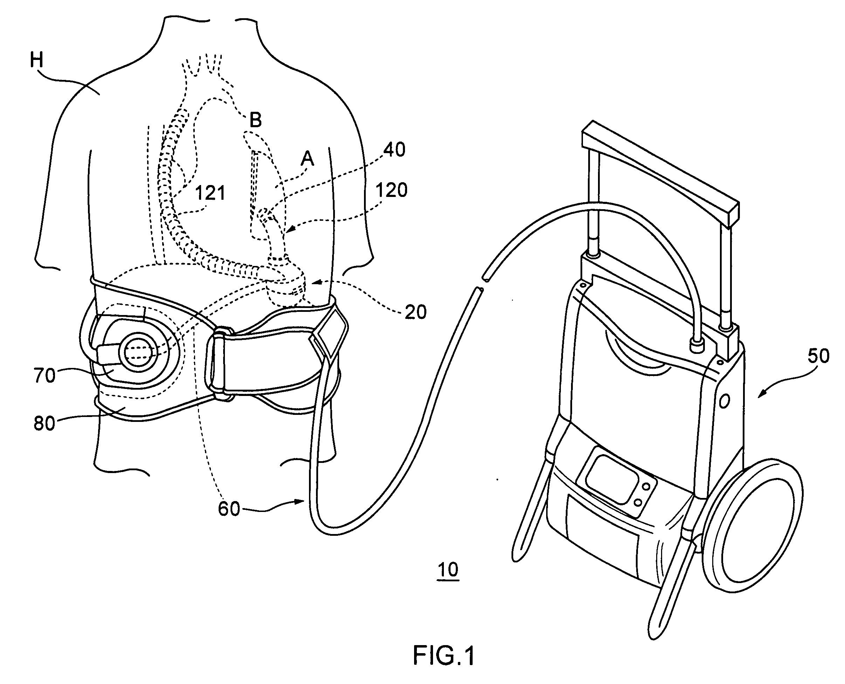

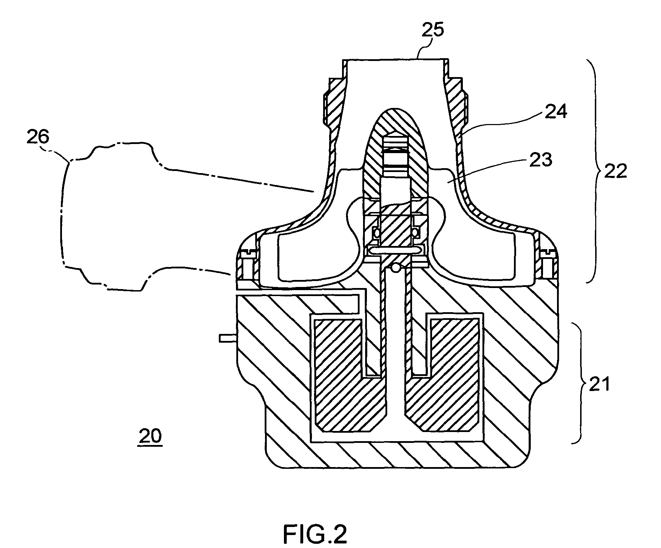

First of all, the blood pump system and the blood pump are explained in conjunction with Fig. 1 and Fig. 2.

Fig. 1 is a view for explaining the blood pump system including the artificial blood vessel system according to an embodiment of the present invention. Fig. 2 is a cross-sectional view showing the blood pump used in the blood pump system shown in Fig. 1.

The blood pump system 10 includes, as shown in Fig. 1, a blood pump 20, artificial blood vessels 120, 121, a cannula 40, an external controller 50 for performing a drive control of the blood pump 20, and a cable 60 which is served for connecting the external controller 50 and the blood pump 20. The blood pump 20 is connected with a left ventricle A of a heart by way of the artificial blood vessel 120 and the cannula 40 and, at the same time, is connected with an aorta B by way of the artificial blood vessel 121.

Here, the detail of the cannula 40 is explained later.

The cable 60 which is connected with the blood pump 20 penetrates a skin of a living body at a portion of a cable fixing tool 70 which is adhered to a belly part of the living body and is led out from the living body and, thereafter, the cable 60 is wrapped around the living body by a half turn or more along a belly belt 80 for cable which is wrapped around the belly part of the living body and, then, the cable 60 is connected with the external controller 50.

The blood pump 20 includes, as shown in Fig. 2, a pump base portion 21 which has a cylindrical motor and a pump portion 22 which is connected with the pump base portion 21. The pump portion 22 includes pump vanes 23 which are driven by way of a rotary shaft of the motor and a pump casing 24 which is connected with the pump base portion 21 such that the pump casing 24 covers the pump vanes 23. That is, the blood pump 20 is configured as follows. Blood in the inside of the left ventricle A of a heart in a human body H flows into the inside of the pump casing 24 through the artificial blood vessel 120 and an inlet opening 25. After being given a flow energy from the pump vanes 23, the blood flows out from the blood pump 20 and flows into the aorta B through an outlet opening 26 formed in a side surface of the pump casing 24 and the artificial blood vessel 121.

Here, although a centrifugal pump is used as the blood pump 20 in the blood pump system 10 according to the embodiment of the present invention, an axial flow pump, a mixed flow pump or a pulsatile pump can be used in place of the centrifugal pump.

Next, the artificial blood vessel system including the artificial blood vessel 120 which connects the blood pump 20 and the left ventricle A of the heart is explained in conjunction with the embodiments of the present invention.

### [Embodiment 1]

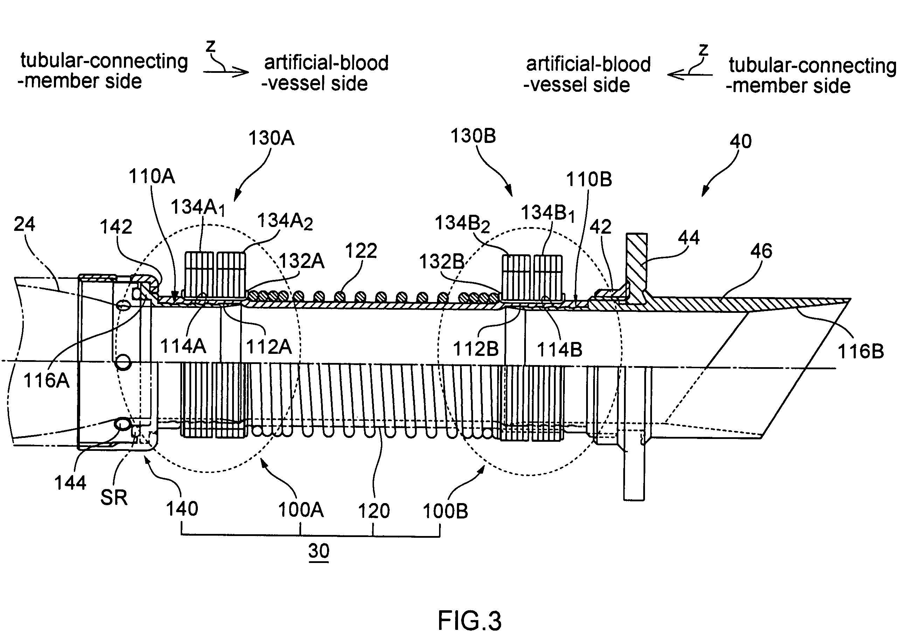

First of all, the artificial blood vessel system according to the embodiment 1 of the present invention is explained in conjunction with Fig. 3 to Fig 5B.

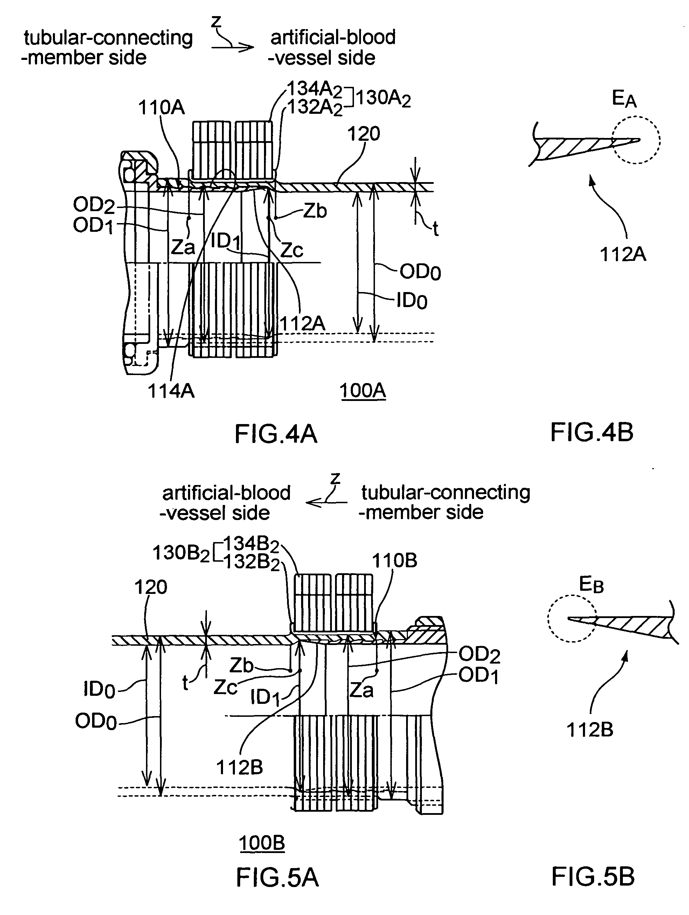

Fig. 3 is a view for explaining the artificial blood vessel system according to the embodiment 1 of the present invention. Fig. 4A and Fig. 4B are views for explaining the artificial blood vessel connecting structure 100A shown in Fig. 3, wherein Fig. 4A is a partial cross-sectional view and Fig. 4B is an enlarged cross-sectional view of an essential part. Fig. 5A and Fig. 5B are views for explaining the artificial blood vessel connecting structure 100B shown in Fig. 3, wherein Fig. 5A is a partial cross-sectional view and Fig. 5B is an enlarged cross-sectional view of an essential part. Here, in Fig. 4A and Fig. 5A, an auxiliary helix is omitted for simplifying the drawings.

The artificial blood vessel system 30 is, as shown in Fig. 3, configured to include the artificial blood vessel 120, the artificial blood vessel connecting structure 100A at the blood pump side, the artificial blood vessel connecting structure 100B at the cannula side, and a connecting ring 140 which is connected with the artificial blood vessel connecting structure 100A and is served for connecting the artificial blood vessel system 30 with the blood pump 20. In the artificial blood vessel system 30, the artificial blood vessel 120 and two tubular connecting members 110A, 110B are connected with each other at these two artificial blood vessel connecting structures 100A, 100B. Since the artificial blood vessel system 30 can obtain the excellent advantageous effects due to the operation of these two artificial blood vessel connecting structures, the artificial blood vessel system 30 is explained in detail by reference with these two artificial blood vessel connecting structures 100A, 100B.

First of all, the artificial blood vessel connecting structure 100A is explained.

The artificial blood vessel connecting structure 100A includes, as shown in Fig. 3 and Fig. 4A, a tubular connecting member 110A, the artificial blood vessel 120, and a connecting assist tool 130A which is served for further strengthening the connection between the tubular connecting member 110A and the artificial blood vessel 120.

Here, the connecting assist tool 130A is configured to bring the artificial blood vessel 120 into pressure contact with the tubular connecting member 110A along the radial direction of the tubular connecting member 110A and, at the same time, brings the artificial blood vessel 120 into pressure contact with the tubular connecting member 110A at the artificial-blood-vessel-side distal end E_{A} (see Fig. 4B) of the tubular connecting member 110A.

Accordingly, in the artificial blood vessel connecting structure 100A, since the connecting assist tool 130A brings the artificial blood vessel 120 into pressure contact with the tubular connecting member 110A along the radial direction of the tubular connecting member 110A, an undesired stress attributed to twisting or the like is not applied to the artificial blood vessel 120. As a result, the connection of the artificial blood vessel 120 becomes more uniform and hence, the reliability of the connection of the artificial blood vessel connecting structure 100A can be enhanced. Further, damage to the artificial blood vessel 120 can be effectively suppressed whereby the lifetime of the artificial blood vessel connecting structure 100A can be prolonged. Further, it is also possible to suppress the adverse influence to the blood pump.

Further, according to the artificial blood vessel connecting structure 100A, since the connecting assist tool 130A also brings the artificial blood vessel 120 into pressure contact with the tubular connecting member 110A at the artificial-blood-vessel-side distal end E_{A} of the tubular connecting member 110A, it is possible to effectively suppress the formation of the minute gap between the artificial blood vessel 120 and the tubular connecting member 110A. As a result, it is possible to effectively suppress the occurrence of the stagnation of the blood flow and, eventually, the occurrence of the thrombus.

In this case, it is considered that, when the artificial blood vessel 120 is bent in the vicinity of the artificial-blood-vessel-side distal end of the tubular connecting member 110A, a minute gap is formed between the artificial blood vessel 120 and the tubular connecting member 110A and eventually the blood flow is stagnated thus considerably increasing the possibility of the occurrence of the thrombus. However, even in such a case, the connecting assist tool 130A brings the artificial blood vessel 120 into pressure contact with the tubular connecting member 110A also at the artificial-blood-vessel-side distal end E_{A} of the tubular connecting member 110A and hence, the formation of the minute gap between the artificial blood vessel 120 and the tubular connecting member 110A can be effectively suppressed. As a result, it is possible to effectively suppress the occurrence of the stagnation of the blood flow and, eventually, the occurrence of the thrombus.

In the artificial blood vessel connecting structure 100A, the connecting assist tool 130A has the property of narrowing an inner diameter thereof. Due to such a constitution, it is possible to bring the artificial blood vessel 120 into pressure contact with the tubular connecting member 110A more uniformly and hence, it is possible to further effectively suppress the applying of the undesired stress attributed to twisting or the like to the artificial blood vessel 120.

In the artificial blood vessel connecting structure 100A, as shown in Fig. 4A, the connecting assist tool 130A satisfies the relationship "Za<Zc and Zb-Zc≥-2×t", wherein assuming an imaginary axis which sets the direction heading for the artificial blood vessel 120 from the tubular connecting member 110A as the normal direction as a z axis, Za are coordinates of the z axis at a tubular-connecting-member-side end of the connecting assist tool 130A, Zb are coordinates of the z axis at an artificial-blood-vessel-side end of the connecting assist tool 130A, Zc are coordinates of the z axis at an artificial-blood-vessel-side distal end E_{A} of the tubular connecting member 110A, and "t" is a wall thickness of an unconnected portion of the artificial blood vessel 120.

To enable the connecting assist tool 130A to surely bring the artificial blood vessel 120 into pressure contact with the tubular connecting member 110A even at the artificial-blood-vessel-side distal end of the tubular connecting member 110A, it is preferable that the position of the artificial-blood-vessel-side end of the connecting assist tool 130A is arranged closer to the artificial-blood-vessel-side than the artificial-blood-vessel-side distal end of the tubular connecting member 110A. However, even when the position of the artificial-blood-vessel-side end of the connecting assist tool 130A is arranged closer to the tubular-connecting-member side than the artificial-blood-vessel-side distal end of the tubular connecting member 110A, with the use of the connecting assist tool 130A which satisfies the relationship "Zb-Zc≥-2×t", the connecting assist tool 130A can bring the artificial blood vessel 120 into pressure contact with the tubular connecting member 110A with a sufficient force even at the artificial-blood-vessel-side distal end of the tubular connecting member 110A and hence, the occurrence of the minute gap between the artificial blood vessel 120 and the tubular connecting member 110A can be suppressed whereby it is possible to effectively suppress the occurrence of the stagnation of the blood flow and, eventually, the occurrence of the thrombus.

In the artificial blood vessel connecting structure 100A, as shown in Fig. 3, an auxiliary helix 122 is wrapped around an outer periphery of the artificial blood vessel 120. Due to such a constitution, it is possible to easily maintain an open state of the artificial blood vessel 120 by making use of the resiliency of the auxiliary helix 122 whereby it is possible to effectively suppress the sharp bending of the artificial blood vessel 120.

The artificial blood vessel connecting structure 100A is also configured such that a relationship "ID₁> ID₀" is satisfied wherein ID₀ is an inner diameter of an unconnected portion of the artificial blood vessel 120, and ID₁ is an inner diameter of the artificial blood vessel 120 at an artificial-blood-vessel-side distal end of the tubular connecting member 110A. Due to such a constitution, at the artificial-blood-vessel-side distal end of the tubular connecting member 110A, the artificial blood vessel 120 is brought into pressure contact with the tubular connecting member 110A even with a resilient force of the artificial blood vessel 120 per se and hence, the formation of the minute gap between the artificial blood vessel 120 and the tubular connecting member 110A can be suppressed whereby it is possible to further effectively suppress the occurrence of the stagnation of the blood flow and, eventually, the occurrence of the thrombus.

The artificial blood vessel connecting structure 100A is preferable that a relationship "ID₁/ID₀<1+0.11×t^{-1/2}" is satisfied wherein t(mm) is a wall thickness of an unconnected portion of the artificial blood vessel 120. Due to such a constitution, the bending of the artificial blood vessel 120 in the vicinity of the artificial-blood-vessel-side distal end of the tubular connecting member 110A can be made gentle and hence, it is possible to suppress the occurrence of the stagnation of the blood flow and, eventually, the occurrence of the thrombus. Here, this relationship is an empirical relationship which is introduced by an experiment carried out by inventors of the present invention and does not always have the physical implication.

The artificial blood vessel connecting structure 100A is configured such that relationships "OD₁>OD₀" and "OD₁>OD₂" are satisfied wherein OD₀ is an outer diameter of an unconnected portion of the artificial blood vessel 120, OD₁ is an outer diameter of a portion which is not subjected to a contact pressure force attributed to the connecting assist tool 130A out of portions of the artificial blood vessel 120 which is applied to an outer periphery of the tubular connecting member 110A, and OD₂ is an outer diameter of a portion which is subjected to the contact pressure force attributed to the connecting assist tool 130A out of the portions of the artificial blood vessel 120 which is applied to the outer periphery of the tubular connecting member 110A. Due to such a constitution, it is guaranteed that the pressure contact force exerted by the connecting assist tool 130A is surely applied to the artificial blood vessel 120. Accordingly, even at the artificial-blood-vessel-side distal end E_{A} of the tubular connecting member 110A, the artificial blood vessel 120 is surely brought into pressure contact with the tubular connecting member 110A and hence, the formation of a minute gap between the artificial blood vessel 120 and the tubular connecting member 110A can be further effectively suppressed whereby the occurrence of the stagnation of the blood flow and, eventually, the occurrence of the thrombus can be further effectively suppressed.

In the artificial blood vessel connecting structure 100A, on the artificial-blood-vessel-side end portion of the tubular connecting member 110A, a sharpened end portion 112A whose wall thickness is gradually decreased from the tubular-connecting-member side to the artificial-blood-vessel side is formed. Due to such a constitution, the flow of the blood at a portion where the artificial blood vessel 120 and the tubular connecting member 110A are connected with each other can be made further smoother and hence, it is possible to further effectively suppress the occurrence of the stagnation of the blood flow and, eventually, the occurrence of the thrombus.

Further, the rounding treatment is applied to the sharpened end portion 112A of the tubular connecting member 110A (see Fig. 4B). Due to such a constitution, a load applied to the artificial blood vessel 120 from the sharpened end portion 112A of the tubular connecting member 110A can be alleviated and hence, an undesired biting of the sharpened end portion 112A of the tubular connecting member 110A into the artificial blood vessel 120 can be obviated. Accordingly, damage to the artificial blood vessel 120 can be effectively suppressed and hence, the lifetime of the artificial blood vessel connecting structure 100A can be further prolonged. Further, it is possible to further effectively suppress the occurrence of the stagnation of the blood flow and, eventually, the occurrence of the thrombus at the artificial-blood-vessel-side distal end of the tubular connecting member 110A. Still further, the assembling operation at the time of connecting the artificial blood vessel 120 with the tubular connecting member 110A can be facilitated.

In the artificial blood vessel connecting structure 100A, an uneven portion 114A is formed on an outer peripheral surface of the tubular connecting member 110A. Due to such a constitution, the artificial blood vessel 120 and the tubular connecting member 110A are connected with each other by way of the uneven portion 114A. Accordingly, it is possible to realize the firm connection between the artificial blood vessel 120 and the tubular connecting member 110A whereby the occurrence of slipping of the artificial blood vessel 120 from the tubular connecting member 110A can be effectively prevented.

The connecting assist tool 130A of the artificial blood vessel connecting structure 100A is, as shown in Fig. 3A, a connecting assist tool which includes two fastening members 134A₁, 134A₂ which are arranged on an outer peripheral surface of the artificial blood vessel 120 by way of a receiving member 132A, wherein the connecting assist tool 130A is arranged on the outer peripheral surface of the artificial blood vessel 120 in a state that a fastening force of the fastening members 134A₁, 134A₂ is applied to the connecting assist tool 130A. Due to such a constitution, the fastening force of the fastening members 134A₁, 134A₂ is transmitted along the radial direction of the tubular connecting member 110A by way of the receiving member 132A. Accordingly, the connecting assist tool 130A brings the artificial blood vessel 120 into pressure contact with the tubular connecting member 110A along the radial direction of the tubular connecting member 110A and hence, an undesired stress attributed to twisting or the like is not applied to the artificial blood vessel 120. As a result, the connection of the artificial blood vessel 120 can be made more uniform and hence, the reliability of the connection between the artificial blood vessel 120 and the tubular connecting member 110A can be further enhanced. Further, damage to the artificial blood vessel 120 can be effectively suppressed and hence, it is possible to further prolong the lifetime of the artificial blood vessel connecting structure 100A.

In the artificial blood vessel connecting structure 100A, as the fastening members 134A₁, 134A₂, a winding member which applies a fastening force in the direction which makes an inner diameter thereof small is used. However, the present invention is not limited to such a winding member and the fastening members 134A₁, 134A₂ made of a heat-shrinkable tube which is liable to shrink in the direction to make an inner diameter thereof small, a shape memory alloy which is liable to shrink in the direction to make an inner diameter thereof small, a tube or a ring made of silicone rubber, a tube or a ring made of fluoric rubber can be also preferably used. Further, fastening members which may be wound around with a tie band, strings or wires may be also used as the fastening members134A₁, 134A₂.

In the artificial blood vessel connecting structure 100A, as the artificial blood vessel 120, an artificial blood vessel made of expanded polytetrafluoroethylene (ePTFE) which exhibits the favorable blood compatibility and the sufficient resiliency is used. Due to such a constitution, it is possible to suppress the occurrence of the thrombus in the inside of the artificial blood vessel 120. Further, it is possible to obtain the favorable connection performance between the artificial blood vessel 120 and the tubular connecting member 110A.

In the artificial blood vessel connecting structure 100A, the tubular connecting member 110A is made of pure titanium which is a material exhibiting a favorable blood compatibility. Due to such a constitution, it is possible to suppress the occurrence of the thrombus in the inside of the tubular connecting member 110A. In place of pure titanium, it is possible to use the titanium alloy (Ti-6Al-4V alloy being particularly favorable).

On a blood contact surface of the artificial blood vessel connecting structure 100A, a coating film made of phospholipid polymer is formed. Due to such a constitution, it is possible to suppress the occurrence of the thrombus on the blood contact surface of the artificial blood vessel connecting structure 100A.

Then, the artificial blood vessel connecting structure 100B is explained hereinafter.

The artificial blood vessel connecting structure 100B includes, as shown in Fig. 3 and Fig. 5A, a tubular connecting member 110B, an artificial blood vessel 120, and a connecting assist tool 130B which is served for firmly connecting the tubular connecting member 110B and the artificial blood vessel 120.

The tubular connecting member 110B is integrally formed with the cannula 40 shown in Fig. 1.

The cannula 40 is, as shown in Fig. 1, a member which allows a portion thereof to face the inside of the left ventricle A and is interposed between the artificial blood vessel 120 which constitutes a portion of the artificial blood vessel system 30 (see Fig. 3) and the heart. The cannula 40 is formed of a cylindrical member which is wholly made of pure titanium or titanium alloy. Due to such a constitution, the cannula 40 can obtain the favorable blood compatibility and hence, the occurrence of the thrombus inside and outside the cannula 40 can be suppressed. Further, as shown in Fig. 3, in the inside of a heart-side opening portion of the cannula 40, a taper portion 116B which has an inner diameter thereof gradually increased from the artificial-blood-vessel side to the heart side is formed. On a middle portion of the cannula 40, a cannula mounting cuff 44 made of felt is mounted using an annular screw 42. The cannula mounting cuff 44 is stitched to the heart.

In the artificial blood vessel connecting structure 100B, the connecting assist tool 130B brings the artificial blood vessel 120 into pressure contact with the tubular connecting member 110B along the radial direction of the tubular connecting member 110B and, at the same time, brings the artificial blood vessel 120 into pressure contact with the tubular connecting member 110B at the artificial-blood-vessel-side distal end E_{B} (see Fig. 5B) of the tubular connecting member 110B.

Accordingly, in the artificial blood vessel connecting structure 100B, in the same manner as the case of the artificial blood vessel connecting structure 100A, since the connecting assist tool 130B brings the artificial blood vessel 120 into pressure contact with the tubular connecting member 110B along the radial direction of the tubular connecting member 110A, an undesired stress attributed to twisting or the like is not applied to the artificial blood vessel 120. As a result, the connection of the artificial blood vessel 120 becomes more uniform and hence, the reliability of the connection of the artificial blood vessel connecting structure 100B can be enhanced. Further, damage to the artificial blood vessel 120 can be effectively suppressed whereby the lifetime of the artificial blood vessel connecting structure 100B can be prolonged.

Further, according to the artificial blood vessel connecting structure 100B, in the same manner as the artificial blood vessel connecting structure 100A, since the connecting assist tool 130B also brings the artificial blood vessel 120 into pressure contact with the tubular connecting member 110B at the artificial-blood-vessel-side distal end E_{B} of the tubular connecting member 110B, it is possible to effectively suppress the formation of the minute gap between the artificial blood vessel 120 and the tubular connecting member 110B. As a result, it is possible to effectively suppress the occurrence of the stagnation of the blood flow and, eventually, the occurrence of the thrombus.

In this case, it is considered that, when the artificial blood vessel 120 is bent in the vicinity of the artificial-blood-vessel-side distal end of the tubular connecting member 110B, a minute gap is formed between the artificial blood vessel 120 and the tubular connecting member 110B and eventually the blood flow is stagnated thus considerably increasing the possibility of the occurrence of the thrombus. However, even in such a case, according to the artificial blood vessel connecting structure 100B, the connecting assist tool 130B brings the artificial blood vessel 120 into pressure contact with the tubular connecting member 110B also at the artificial-blood-vessel-side distal end E_{B} of the tubular connecting member 110B and hence, the formation of the minute gap between the artificial blood vessel 120 and the tubular connecting member 110B can be effectively suppressed. As a result, it is possible to effectively prevent the occurrence of the stagnation of the blood flow and, eventually, the occurrence of the thrombus.

In the artificial blood vessel connecting structure 100B, the connecting assist tool 130B has the property of narrowing an inner diameter thereof. Due to such a constitution, it is possible to bring the artificial blood vessel 120 into pressure contact with the tubular connecting member 110B more uniformly and hence, it is possible to further effectively prevent the applying of the undesired stress attributed to twisting or the like to the artificial blood vessel 120.

Here, in the artificial blood vessel connecting structure 100B, the tubular connecting member 110B and the cannula 40 are integrally formed. However, the present invention is not limited to such a constitution, and the tubular connecting member 110B and the cannula 40 may be formed separately.

As described above, the artificial blood vessel system 30 according to the embodiment 1 is the artificial blood vessel system which exhibits the prolonged lifetime, has high reliability in connection and can effectively suppress the occurrence of the thrombus and, at the same time, can be easily connected with the blood pump. Accordingly, by incorporating such an excellent artificial blood vessel system 30 into the blood pump, at the time of embedding the blood pump into the living body, it is possible to obtain the excellent blood pump system which can reduce a burden imposed on an operator at the time of embedding the blood pump into a living body and can suppress the occurrence of the thrombus after embedding the blood pump into the living body.

### [Embodiment 2]

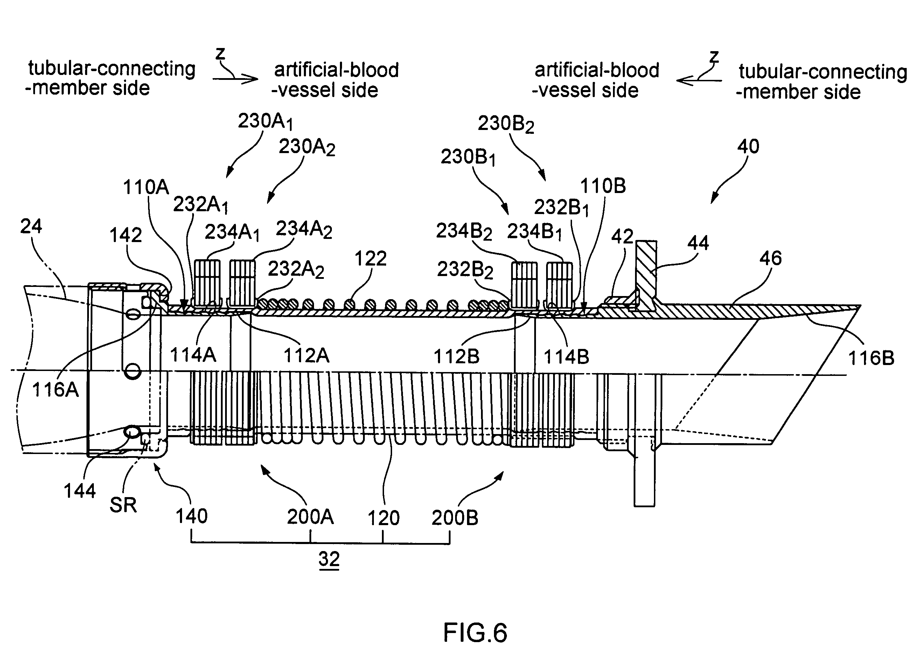

The embodiment 2 according to the present invention is explained in conjunction with Fig. 6.

Fig. 6 is a view for explaining an artificial blood vessel system according to the embodiment 2 of the present invention. In Fig. 6, parts which are identical with the parts shown in Fig. 3 are given the same symbols and their detailed explanation is omitted.

The artificial blood vessel system 32 according to the embodiment 2 differs from the artificial blood vessel system 30 according to the embodiment 1 in the configuration and the number of the connecting assist tools. That is, in the artificial blood vessel system 30 according to the embodiment 1, one connecting assist tool 130A having one receiving member 132A which is relatively long in the z direction and two fastening members 134A₁, 134A₂ is used (in case of the artificial blood vessel connecting structure 100A). The same goes for the artificial blood vessel connecting structure 100B.

On the other hand, in the artificial blood vessel system 32 according to the embodiment 2, two connecting assist tools 230A₁, 230A₂ including two receiving members 232A₁, 232A₂ which are relatively short in the z direction and two fastening members 234A₁, 234A₂ which are arranged corresponding to these two receiving members 232A₁, 232A₂ respectively are used (in case of the artificial blood vessel connecting structure 200A). The same goes for the artificial blood vessel connecting structure 200B.

In this manner, although the artificial blood vessel system 32 is provided with the connecting assist tools 230A₁, 230A₂ having the configuration and the number different from the connecting assist tool in the artificial blood vessel system 30, in the same manner as the artificial blood vessel system 30, these connecting assist tools 230A₁, 230A₂ function so as to bring the artificial blood vessel 120 into pressure contact with the tubular connecting member 110A along the radial direction of the tubular connecting member 110A and, at the same time, at the artificial-blood-vessel-side distal end of the tubular connecting member 110A.

This is because that the connecting assist tools 230A₁, 230A₂ are cooperatively operated so as to bring the artificial blood vessel 120 into pressure contact with the tubular connecting member 110A along the radial direction of the tubular connecting member 110A and, at the same time, the connecting assist tool 230A₂ out of the connecting assist tools 230A₁, 230A₂ functions so as to bring the artificial blood vessel 120 into pressure contact with the tubular connecting member 110A at the artificial-blood-vessel-side distal end of the tubular connecting member 110A.

Accordingly, also in the artificial blood vessel system 32, the substantially same advantageous effects as those of the artificial blood vessel system 30 can be obtained.

### [Embodiment 3]

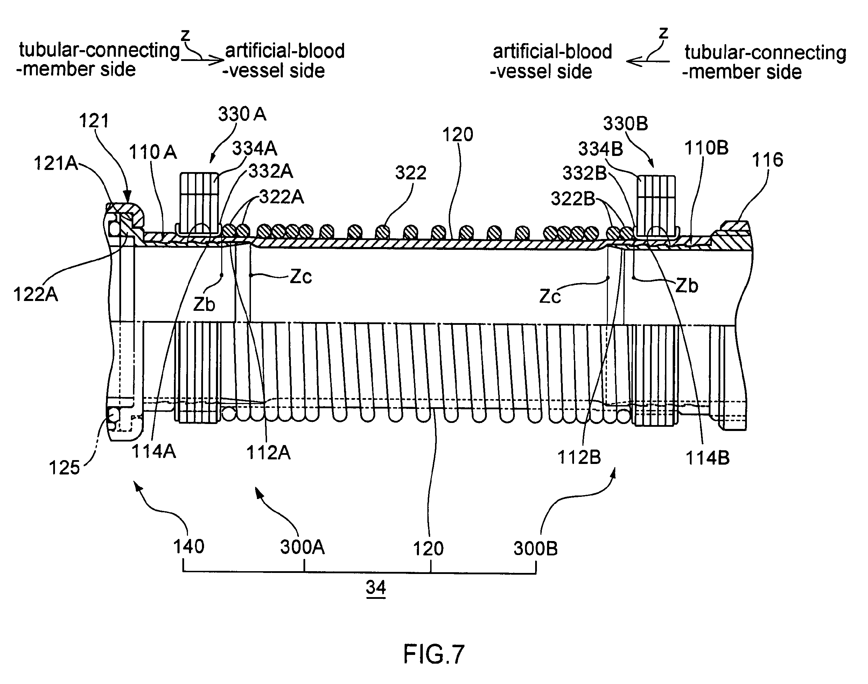

The embodiment 3 of the present invention is explained in conjunction with Fig. 7.

Fig. 7 is a view for explaining an artificial blood vessel system according to the embodiment 3 of the present invention. In Fig. 7, parts which are identical with the parts shown in Fig. 3 are given the same symbols and their detailed explanation is omitted.

The artificial blood vessel system 34 according to the embodiment 3 differs from the artificial blood vessel system 32 according to the embodiment 2 in the constitution of the connecting assist means. That is, in the artificial blood vessel system 32 according to the embodiment 2, as the constitution of the connecting assist means, two connecting assist tools 230A₁, 230A₂ including two receiving members 232A₁, 232A₂ which are relatively short in the z direction and two fastening members 234A₁, 234A₂ which are arranged corresponding to these two receiving members 232A₁, 232A₂ respectively are used (in case of the artificial blood vessel connecting structure 200A). The same goes for the artificial blood vessel connecting structure 200B.

On the other hand, in the artificial blood vessel system 34 according to the embodiment 3, as the constitution of the connecting assist means, connecting assist means which is constituted of a connecting assist tool 330A including one receiving member 332A which is relatively short in the z direction and one fastening member 334A which is arranged corresponding to this receiving member 332A and a connecting assist portion 322A which forms a portion of the auxiliary helix 322 which is arranged close to the contact portion with the connecting assist tool 330A is used (in case of the artificial blood vessel connecting structure 300A). The same goes for the artificial blood vessel connecting structure 300B.

In this manner, the artificial blood vessel system 34 is provided with the connecting assist means having the constitution different from the constitution of the artificial blood vessel system 32 (the connecting assist tool 330A and the connecting assist portion 322A which is formed of the portion the auxiliary helix). In the same manner as the artificial blood vessel system 32, these connecting assist means (the connecting assist tool 330A and the connecting assist portion 322A which is formed of the portion the auxiliary helix) function so as to bring the artificial blood vessel 120 into pressure contact with the tubular connecting member 110A along the radial direction of the tubular connecting member 110A and, at the same time, at the artificial-blood-vessel-side distal end of the tubular connecting member 110A.

This is because that the connecting assist tools 330A and the connecting assist portion 322A which is formed of the portion the auxiliary helix are cooperatively operated so as to bring the artificial blood vessel 120 into pressure contact with the tubular connecting member 110A along the radial direction of the tubular connecting member 110A and, at the same time, the connecting assist portion 322A which is formed of the portion the auxiliary helix functions so as to bring the artificial blood vessel 120 into pressure contact with the tubular connecting member 110A at the artificial-blood-vessel-side distal end of the tubular connecting member 110A.

In this manner, in the artificial blood vessel connecting structure 300A, the connecting assist tool 330A is configured to satisfy the relationship "Zb<Zc", wherein assuming an imaginary axis which sets the direction heading for the artificial blood vessel 120 from the tubular connecting member 110A as the normal direction as a z axis, Zb are coordinates of the z axis at an artificial-blood-vessel-side end of the connecting assist tool 330A, and Zc are coordinates of the z axis at an artificial-blood-vessel-side distal end of the tubular connecting member 110A. Accordingly, although the connecting assist tool 330A when used in a single form does not perform a function of bringing the artificial blood vessel 120 into pressure contact with the tubular connecting member 110A at the artificial-blood-vessel-side distal end of the tubular connecting member 110A, the connecting assist portion 322A which is formed of the portion the auxiliary helix functions so as to bring the artificial blood vessel 120 into pressure contact with the tubular connecting member 110A at the artificial-blood-vessel-side distal end of the tubular connecting member 110A

The same goes for the artificial blood vessel connecting structure 300B.

Accordingly, in the artificial blood vessel system 34 of the embodiment 3 also, the substantially same advantageous effects as the artificial blood vessel system 30 of the embodiment 1 or artificial blood vessel system 32 of the embodiment 2 can be obtained.

As has been described heretofore, by using the respective artificial blood vessel systems according to the embodiments 1 to 3, the reliability of connection between the artificial blood vessel and the tubular connecting member in the artificial blood vessel system can be enhanced. Further, the damage to the artificial blood vessel can be effectively suppressed and the lifetime of the artificial blood vessel system can be prolonged. Still further, the occurrence of the stagnation of the blood flow and, eventually, the occurrence of the thrombus can be effectively suppressed. Still further, even when the artificial blood vessel is bent in the vicinity of the artificial-blood-vessel-side distal end of the tubular connecting member, the occurrence of the stagnation of the blood flow and, eventually, the occurrence of the thrombus can be effectively suppressed.

### [Embodiment 4]

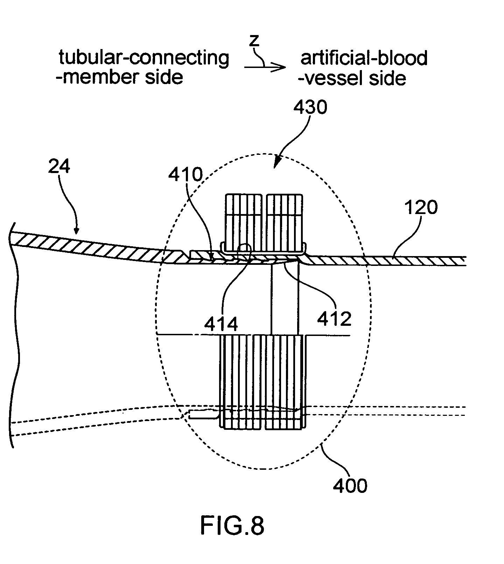

Fig. 8 is a view showing the connection portion (artificial blood vessel connecting structure) between the blood pump and the artificial blood vessel used in the blood pump system according to the embodiment 4. Here, in Fig. 8, to simplify the view, the auxiliary helix is omitted.

The blood pump system according to the embodiment 4 is, as shown in Fig. 8, a blood pump system which includes a blood pump having a tubular connecting member 410 at a suction-side distal end portion of the pump casing 24, an artificial blood vessel 120 which is connected with the tubular connecting member 410, and a connecting assist tool 430 for strengthening the connection between the artificial blood vessel 120 and the tubular connecting member 410. The connecting assist tool 430 is, in the same manner as the embodiment 1, constituted so as to bring the artificial blood vessel 120 into pressure contact with the tubular connecting member 410 along the radial direction of the tubular connecting member 410 and, at the same time, at the artificial blood vessel side distal end of the tubular connecting member 410.

Accordingly, the blood pump system according to the embodiment 4 is provided with the artificial blood vessel connecting structure 400 which can prolong the lifetime and can exhibit the high connection reliability and, further, can effectively suppress the occurrence of the thrombus. Accordingly, this embodiment 4 also can provide the highly reliable excellent blood pump system which can suppress the occurrence of the thrombus.

Here, the embodiments 1 to 4 have been explained with respect to the case in which the artificial blood vessel 120 is connected to the suction side of the blood pump as an example. However, the present invention is not limited to such a constitution and the substantially equal advantageous effects can be obtained with respect to a case in which the artificial blood vessel 120 is connected to the discharge side of the blood pump.

The present invention provides an artificial blood vessel system which can suppress the occurrence of the stagnation of a blood flow and, eventually, the occurrence of the thrombus, can enhance the reliability of the connection, and can prolong the lifetime of the artificial blood connecting structure. In the artificial blood vessel system which includes an artificial blood vessel, a tubular connecting member which is connected with the artificial blood vessel, and connecting assist means for strengthening the connection between the artificial blood vessel and the tubular connecting member, the connecting assist means is configured to be capable of bringing the artificial blood vessel into pressure contact with the tubular connecting member not only along the radial direction of the tubular connecting member but also at an artificial-blood-vessel-side distal end of the tubular connecting member.

## Claims

1. An artificial blood vessel system comprising:
an artificial blood vessel;
a tubular connecting member which is connected with the artificial blood vessel; and
connecting assist means for strengthening the connection between the artificial blood vessel and the tubular connecting member,
wherein the connecting assist means is configured to be capable of bringing the artificial blood vessel into pressure contact with the tubular connecting member not only along the radial direction of the tubular connecting member but also at an artificial-blood-vessel-side distal end of the tubular connecting member.

2. An artificial blood vessel system according to claim 1,
wherein the connecting assist means has the property of narrowing an inner diameter thereof.

3. An artificial blood vessel system according to claim 1 or 2,
wherein the connecting assist means is constituted of a connecting assist tool which satisfies a following relationship (a):
(a) the relationship "Za<Zc and Zb-Zc≥-2×t", wherein assuming an imaginary axis which sets the direction heading for the artificial blood vessel from the tubular connecting member as the normal direction as a z axis, Za are coordinates of the z axis at a tubular-connecting-member-side end of the connecting assist tool, Zb are coordinates of the z axis at an artificial-blood-vessel-side end of the connecting assist tool, Zc are coordinates of the z axis at an artificial-blood-vessel-side distal end of the tubular connecting member, and t is a wall thickness of an unconnected portion of the artificial blood vessel.

4. An artificial blood vessel system according to claim 3,
wherein a member which prevents the artificial blood vessel from being sharply bent is mounted on an outer periphery of the artificial blood vessel.

5. An artificial blood vessel system according to claim 1 or 2,
wherein a member for preventing the artificial blood vessel from being sharply bent is mounted on an outer periphery of the artificial blood vessel, and
the connecting assist means is constituted of a connecting assist tool which satisfies a following relationship (b) and the member for preventing the artificial blood vessel from being sharply bent, and the tubular-connecting-member-side end of the member for preventing the artificial blood vessel from being sharply bent is brought into contact with the artificial-blood-vessel-side end of the connecting assist tool:
(b) the relationship "Zb<Zc", wherein assuming an imaginary axis which sets the direction heading for the artificial blood vessel from the tubular connecting member as the normal direction as a z axis, Zb are coordinates of the z axis at an artificial-blood-vessel-side end of the connecting assist tool, and Zc are coordinates of the z axis at an artificial-blood-vessel-side distal end of the tubular connecting member.

6. An artificial blood vessel system according to any one of preceding claims 1 to 5,
wherein a relationship "ID₁≥ ID₀" is satisfied wherein ID₀ is an inner diameter of an unconnected portion of the artificial blood vessel and ID₁ is an inner diameter of the artificial blood vessel at an artificial-blood-vessel-side distal end of the tubular connecting member.

7. An artificial blood vessel system according to claim 6,
wherein a relationship "ID₁/ID₀<1+0.11×t^{-1/2}" is satisfied wherein t(mm) is a wall thickness of an unconnected portion of the artificial blood vessel.

8. An artificial blood vessel system according to any one of preceding claims 1 to 7,
wherein relationships "OD₁>OD₀" and "OD₁>OD₂" are satisfied wherein OD₀ is an outer diameter of an unconnected portion of the artificial blood vessel, OD₁ is an outer diameter of a portion which is not subjected to a contact pressure force attributed to the connecting assist means out of portions of the artificial blood vessel which is applied to an outer periphery of the tubular connecting member, and OD₂ is an outer diameter of a portion which is subjected to the contact pressure force attributed to the connecting assist means out of the portions of the artificial blood vessel which is applied to the outer periphery of the tubular connecting member.

9. An artificial blood vessel system according to any one of preceding claims 1 to 8,
wherein on the artificial-blood-vessel-side end portion of the tubular connecting member, a sharpened end portion whose wall thickness is gradually decreased from the tubular-connecting-member side to the artificial-blood-vessel side is formed.

10. An artificial blood vessel system according to claim 9,
wherein the rounding treatment is applied to the sharpened end portion of the tubular connecting member.

11. An artificial blood vessel system according to any one of preceding claims 1 to 10,
wherein an uneven portion is formed on an outer peripheral surface of the tubular connecting member.

12. An artificial blood vessel system according to any one of preceding claims 3 to 5,
wherein the connecting assist tool is a connecting assist tool which includes a fastening member arranged on an outer peripheral surface of the artificial blood vessel by way of a receiving member, and the connecting assist tool is arranged on an outer peripheral surface of the artificial blood vessel in a state that a fastening force of the fastening member is applied to the connecting assist tool.

13. An artificial blood vessel system according to any one of preceding claims 1 to 12,
wherein the artificial blood vessel is made of a material which exhibits a favorable blood compatibility.

14. An artificial blood vessel system according to any one of preceding claims 1 to 13,
wherein the tubular connecting member is made of a material which exhibits a favorable blood compatibility.

15. An artificial blood vessel system according to any one of preceding claims 1 to 14,
wherein on a blood contact surface of the artificial blood vessel system, a coating film made of a material having blood compatibility and antithrombogenicity is formed.

16. An artificial blood vessel system according to any one of preceding claims 1 to 15,
wherein the artificial blood vessel system further includes a connecting ring for connecting the artificial blood vessel to the blood pump.

17. An artificial blood vessel system according to any one of preceding claims 1 to 16,
wherein the artificial blood vessel system further includes a cannula for connecting the artificial blood vessel to the heart.

18. A connecting assist tool for strengthening the connection between an artificial blood vessel and a tubular connecting member,
wherein the connecting assist tool is served for the artificial blood vessel system according to claim 3, 4, 5 or 12.

19. A pump system comprising a blood pump and an artificial blood vessel system which is connected to at least one side out of a suction side and a discharge side of the blood pump,
wherein the artificial blood vessel system is the artificial blood vessel system according to claim 16.

20. A pump system comprising:
a blood pump which includes a tubular connecting member having at least one side out of a suction side and a discharge side thereof;
an artificial blood vessel which is connected with the tubular connecting member, and
connecting assist means which is served for strengthening the connection between the artificial blood vessel and the tubular connecting member,
wherein the connecting assist means is configured to be capable of bringing the artificial blood vessel into pressure contact with the tubular connecting member not only in the radial direction of the tubular connecting member but also at an artificial-blood-vessel-side distal end of the tubular connecting member.
